# EUROPEAN PATENT APPLICATION

(11) **EP 4 802 960 A1**
(43) Date of publication of application: **09.09.2026**
(21) Application number: 24910732.7
(22) Date of filing: 11.12.2024
(51) Int. Cl.: A47C 31/00, A61F 5/56

(54) **SMART BED CONTROL METHOD AND SYSTEM, AND SMART BED**

(30) Priority: 30.12.2023 CN 202311873042
(71) Applicant: DewertOkin Technology Group Co., Ltd., Jiaxing, Zhejiang 314031 (CN)
(72) Inventor: MEI, Weifeng, Jiaxing, Zhejiang 314031 (CN); DONG, Wenli, Jiaxing, Zhejiang 314031 (CN); LV, Kai, Jiaxing, Zhejiang 314031 (CN); ZHANG, Xichen, Jiaxing, Zhejiang 314031 (CN)
(74) Representative: Studio Torta S.p.A.
(86) International application number: PCT/CN2024/138591
(87) International publication number: WO 2025/139801

(57) **Abstract**

Disclosed are a method and system for controlling a smart bed, and a smart bed. The method for controlling the smart bed includes: collecting, by a sound signal collection device, N groups of breath sound signals emitted by an object at every preset time interval, where the object is on the smart bed (S102); detecting, by a snore detection device, whether snore signals exist in the N groups of breath sound signals (S104); and controlling the smart bed to perform a snore-stopping action in a case that the number of groups of breath sound signals including the snore signals in the N groups of breath sound signals is detected to be greater than M, where M is an integer less than or equal to N (S106).

## Description

### Cross-Reference to Related Application

The present disclosure claims the priority to Chinese Patent Application No. 202311873042.1, filed on December 30, 2023 and entitled "Method and system for controlling smart bed, and smart bed", which is incorporated in its entirety or as a part herein by reference.

### Technical Field

The present disclosure relates to the field of smart home, and particularly relates to a method and system for controlling a smart bed, and a smart bed.

### Background

Snoring is a common sleep disorder that affects sleep quality and causes a poor mental state of a snorer. In severe cases, the snorer may undergo sleep apnea, thus affecting physical health. Voice-based snoring monitoring is a technology for detecting and recording snoring sounds with a voice chip and related software. However, smart beds in the related art generally do not have a snore-stopping function, resulting in poor user experience and sleep quality.

No effective solution has been put forward yet at present to solve the problems.

### Summary

Examples of the present disclosure provide a method and system for controlling a smart bed, and a smart bed, to solve at least the technical problem that smart beds in the related art do not have a snore-stopping function, resulting in poor user experience and sleep quality.

One aspect of the examples of the present disclosure provides a method for controlling a smart bed. The method includes: collecting, by a sound signal collection device, N groups of breath sound signals emitted by an object at every preset time interval, where the object is on the smart bed; detecting, by a snore detection device, whether snore signals exist in the N groups of breath sound signals; and controlling the smart bed to perform a snore-stopping action in a case that the number of groups of breath sound signals including the snore signals in the N groups of breath sound signals is detected to be greater than M, M is an integer less than or equal to N.

Another aspect of the examples of the present disclosure further provides a system for controlling a smart bed. The system includes: a sound signal collection device, a main control device, and a snore detection device. The sound signal collection device and the snore detection device are separately connected to the main control device. The main control device is configured to perform the method for controlling the smart bed according to any one of the examples.

Yet another aspect of the examples of the present disclosure further provides a smart bed. The smart bed includes a bed body, and the system for controlling a smart bed. The system for controlling a smart bed is arranged in the bed body.

Still another aspect of the examples of the present disclosure further provides an electronic device. The electronic device includes one or more processors and a memory. The memory is configured to store one or more programs. When the one or more programs are executed by the one or more processors, the one or more processors are caused to implement the method for controlling the smart bed according to any one of the examples.

In the examples of the present disclosure, the sound signal collection device collects the N groups of breath sound signals emitted by the object at every preset time interval, where the object is on the smart bed; the snore detection device detects whether the snore signals exist in the N groups of breath sound signals; and the smart bed is controlled to perform the snore-stopping action in a case that the number of groups of breath sound signals including the snore signals in the N groups of breath sound signals is detected to be greater than M, M is an integer less than or equal to N. In this way, smart bed control and snore stopping are implemented based on the snore signals of a user. Thus, technical effects of effective snore stopping of the user through control of the smart bed, improvement in sleep quality of the user, and better user experience are achieved. In addition, the technical problem that smart beds in the related art do not have a snore-stopping function, resulting in poor user experience and sleep quality is solved.

### Brief Description of the Drawings

The accompanying drawings described herein serve to provide a further understanding of the present disclosure and constitute part of the present disclosure, and illustrative examples of the present disclosure and the description thereof serve to explain the present disclosure and are not to be construed as unduly limiting the present disclosure. In the drawings:
Fig. 1 is a flowchart of a method for controlling a smart bed according to an example of the present disclosure;
Fig. 2 is a schematic structural diagram of a system for controlling a smart bed according to an example of the present disclosure;
Fig. 3 is a schematic structural diagram of an optional system for controlling a smart bed according to an example of the present disclosure;
Fig. 4 is a schematic structural diagram of another optional system for controlling a smart bed according to an example of the present disclosure; and
Fig. 5 is a schematic diagram of an apparatus for controlling a smart bed according to an example of the present disclosure.

### Detailed Description of the Embodiments

In order to make those skilled in the art better understand a solution of the present disclosure, the technical solution in examples of the present disclosure will be described below clearly and comprehensively in conjunction with accompanying drawings in the examples of the present disclosure. Apparently, the examples described are merely some examples rather than all examples of the present disclosure. Based on the examples of the present disclosure, all other examples obtained by those of ordinary skill in the art without making creative efforts shall fall within the protection scope of the present disclosure.

It should be noted that the terms such as "first" and "second" in the description and claims of the present disclosure and in the above drawings are used to distinguish between similar objects and not necessarily to describe a particular order or sequential order. It should be understood that data used in this way can be interchanged in appropriate cases, such that the examples of the present disclosure described herein can be implemented in a sequence other than those illustrated or described herein. Moreover, the terms "include", "comprise", "have", and any variations thereof are intended to cover non-exclusive inclusion. For instance, processes, methods, systems, products or devices including a series of steps or units do not need to be limited by the steps or units explicitly listed, and may include other steps or units not explicitly listed or inherent to the processes, methods, products, or devices.

An example of the present disclosure provides an example of a method for controlling a smart bed. It should be noted that the steps illustrated in flowcharts of the accompanying drawings may be performed in a computer system such as a set of computer-executable instructions, and although a logical order is illustrated in the flowcharts, in some cases, the steps shown or described may be performed in an order different from that herein.

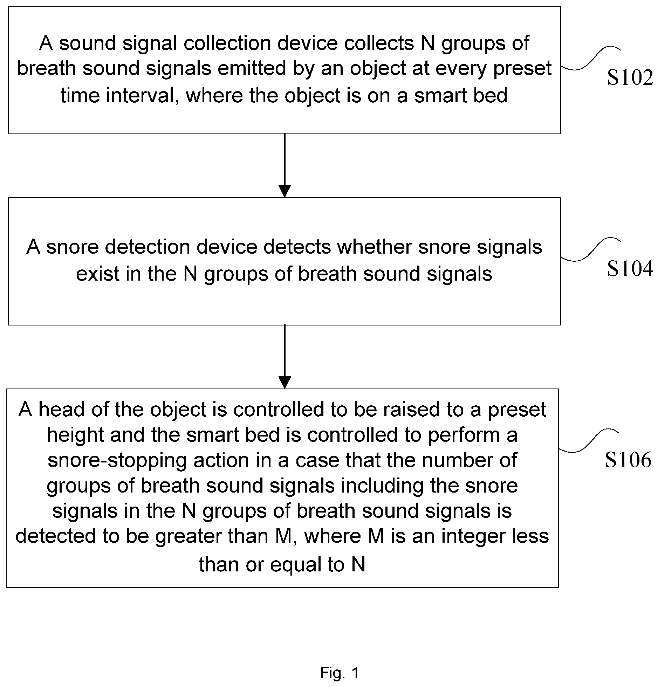

Fig. 1 is a flowchart of a method for controlling a smart bed according to an example of the present disclosure. As shown in Fig. 1, the method includes the following steps:
S102, a sound signal collection device collects N groups of breath sound signals emitted by an object at every preset time interval, wherein the object is on the smart bed.

As at least one alternative embodiment, the sound signal collection device may be an offline voice chip, and is configured to collect a group of breath sound signals at every preset time interval (for instance, every 1 minute). Then, whether to enable a snore-stopping function is determined based on the collected N groups (for instance, 5 groups) of breath sound signals.

In an optional example, the step that the sound signal collection device collects the N groups of breath sound signals emitted by the object at every preset time interval includes the following steps: time during which the object is on the smart bed is determined in a case that the object is detected to be on the smart bed; whether the time during which the object is on the smart bed falls within a preset period is detected; and the sound signal collection device collects the N groups of breath sound signals emitted by the object at every preset time interval in a case that the time during which the object is on the smart bed is detected to fall within the preset period.

As at least one alternative embodiment, before the breath sound signals are collected, whether the object (i.e., a user) is on the bed and whether the object is currently in a sleep state need to be determined. A pressure sensor may be arranged on a mattress of the smart bed, and whether the object is currently on the smart bed, that is, whether there is a person on the bed, may be determined according to collected pressure signals. Or, in a case that the time during which the object is on the bed falls within the preset period (for instance, from 11 p.m. to 7 a.m.), it is determined that the user is in a sleep state. In this case, the sound signal collection device is activated to start collection of the breath sound signals emitted by the object.

It should be noted that the preset period may be independently set according to sleep habits of the user. For instance, if the user is accustomed to sleeping from 11 p.m. to 7 a.m., the preset period is set as 11 p.m. to 7 a.m. If the user is accustomed to sleeping from 10 p.m. to 6 a.m., the preset period is set as 10 p.m. to 6 a.m. In this way, merely needing a pressure sensor or time detection, whether to activate the sound signal collection device or not is able to be determined, leading to lower device investment cost. In addition, periodic activation of the sound signal collection device is able to reduce a startup frequency of the device, thus reducing energy consumption.

In an optional example, the step that the sound signal collection device collects the N groups of breath sound signals emitted by the object at every preset time interval includes the following steps: an in-bed state and sleep state data of the object are detected based on a flexible piezoelectric sensor; and the sound signal collection device collects the N groups of breath sound signals emitted by the object at every preset time interval in a case that the in-bed state indicates that the object is on the smart bed and the sleep state data indicates that the object is in a sleep state.

As at least one alternative embodiment, before the sound signals are collected, whether the object (i.e., the user) is on the bed and whether the object is currently in the sleep state need to be determined. The flexible piezoelectric sensor is able to be arranged to monitor the in-bed state and the sleep state data of the object. Based on the monitored in-bed state and sleep state data of the object, the sleep state of the object may be accurately determined, and the sound signal collection device is able to be precisely controlled to be turned on or off. In this way, energy consumption is reduced, and meanwhile, an operating frequency of the device is reduced, and further wear and tear of the device are reduced.

S104, a snore detection device detects whether snore signals exist in the N groups of breath sound signals.

As at least one alternative embodiment, the snore detection device may be a snore monitoring chip. The snore monitoring chip and the offline voice chip may be arranged on a same circuit board, so as to reduce actual occupied space of device components.

S106, the smart bed is controlled to perform a snore-stopping action in a case that the number of groups of breath sound signals including the snore signals in the N groups of breath sound signals is detected to be greater than M, wherein M is an integer less than or equal to N.

As at least one alternative embodiment, in a case that the snore signals are detected to exist in at least M groups of breath sound signals in the N groups of breath sound signals, the snore-stopping function is activated.

As at least one alternative embodiment, the snore-stopping action at least includes raising a head of the object to a preset height, and may further include controlling the object to turn over. The preset height may be independently set by the user according to sleep habits. The snore-stopping action is able to implement automatic snore stopping without affecting sleep of the object.

As at least one alternative embodiment, the snore-stopping action may be implemented by driving the smart bed to move through a drive motor, or by controlling inflation and deflation of an air bag through an inflation system arranged on the smart bed. For instance, if the snore-stopping action is controlling the head of the object to be raised to the preset height, the head of the object may be controlled to be raised by directly controlling a predetermined part (i.e., a part corresponding to the head) of the smart bed to be raised. Or, the head of the object may be raised by triggering the air bag, and a sleeping posture of the object is adjusted through automatic raising of the head of the object, thus implementing snore stopping.

As at least one alternative embodiment, the processes of collecting the breath sound signals and detecting snores are real-time processes. The obtained N groups of breath sound signals are dynamically updated over time. For instance, if the time interval is 1 minute and N is 5, 5 groups of breath sound signals collected in the latest 5 minutes are obtained, and snore signals in the 5 groups of breath sound signals are detected. If the snore signals exist in 3 or more groups of breath sound signals in the 5 groups of breath sound signals, it is determined that the snore-stopping function is activated, and the head of the object is controlled to be raised to a certain height.

It should be noted that the user may only snore transiently during sleep, and for instance, snore once or twice and then stop snoring. Thus, in a snore detection process, detection of the snore signals is performed many times by obtaining consecutive groups of breath sound signals, such that the snore signals are accurately identified, and frequent activation of relevant devices (such as the sound signal collection device and the snore detection device) caused by transient snoring of the user is avoided.

In an optional example, after the smart bed is controlled to perform the snore-stopping action in a case that the number of groups of breath sound signals including the snore signals in the N groups of breath sound signals is detected to be greater than M, the method further includes the following steps: whether snore signals exist in breath sound signals collected by the sound signal collection device is continuously detected one or more times, and in a case that results of one or more times of repeated detection indicate that the snore signals exist in the breath sound signals, the head of the object is continuously controlled to be further raised one or more times until no snore signals are detected in the breath sound signals collected by the sound signal collection device.

As at least one alternative embodiment, in order to avoid snore-stopping failure after the smart bed is raised, the sound signal collection device may continuously collect the breath sound signals of the object after the smart bed is raised, and whether the snore signals exist in the collected breath sound signals may be detected. If no snore signals are detected, it indicates that snore stopping is successful. If the snore signals still exist in the newly collected breath sound signals, the smart bed is further controlled to be raised until no snore signals are detected in the breath sound signals collected by the sound signal collection device.

As at least one alternative embodiment, after the smart bed is raised, during continuous collection of the breath sound signals of the object through the sound signal collection device, whether to further control the head of the object to be raised may be determined by collecting K groups (for instance, 5 groups) of breath sound signals and detecting whether snore signals exist in M or more groups (for instance, 3 groups) of breath sound signals in the obtained K groups of breath sound signals. K is an integer greater than or equal to 1, and M is an integer less than or equal to K.

In an optional example, the method further includes the following steps: a cumulative raising frequency of the head of the object is determined; whether the cumulative raising frequency reaches a preset raising frequency is detected; and the head of the object is controlled to be restored to an initial height state in a case that the cumulative raising frequency reaches the preset raising frequency, and the head of the object is controlled to be raised again from the initial height state.

As at least one alternative embodiment, a cumulative raising upper limit of the head of the object is set. In a case that the cumulative raising frequency of the head of the object reaches an upper limit (i.e., the preset raising frequency), the head of the object is restored to the initial height state. The initial height state may be a fully-flat state or a height state preset by the user. This mode may prevent sleep quality from being affected by excessive raising of the head of the object.

As at least one alternative embodiment, when the head of the object is restored to the initial height state, the cumulative raising frequency is reset to zero, and counting of the cumulative raising frequency is restarted from the initial height state.

In an optional example, the steps that the head of the object is controlled to be restored to the initial height state in a case that the cumulative raising frequency reaches the preset raising frequency, and the head of the object is controlled to be raised again from the initial height state include the following steps: a cumulative restoration frequency of the head of the object is determined in a case that the cumulative raising frequency reaches the preset raising frequency, where the cumulative restoration frequency is configured to indicate a frequency of restoring the head of the object to the initial height state; and the head of the object is controlled to be restored to the initial height state in a case that the cumulative restoration frequency does not reach a preset restoration frequency, and the head of the object is controlled to be raised again from the initial height state.

As at least one alternative embodiment, after the cumulative raising frequency of the head of the object reaches the preset raising frequency, whether the cumulative restoration frequency of the head of the object reaches the preset restoration frequency is further determined. If no, the head of the object may continuously be restored to the initial height state, and the head of the object may be controlled to be raised again. The user may log in to a corresponding target application to customize the preset raising frequency and the preset restoration frequency. The above mode may prevent frequent head raising from affecting sleep quality of the user.

In an optional example, the method further includes the following step: collection of the breath sound signals emitted by the object is stopped in a case that the cumulative restoration frequency reaches the preset restoration frequency.

As at least one alternative embodiment, if a snore-stopping effect is still not achieved after repeated raising and restoration operations, collection and further raising are stopped, and collection of the breath sound signals emitted by the object is terminated. For instance, when snoring is detected, the head is raised by one level. If snoring is still detected, the head is raised by another level, with a maximum of three raising levels in total. If snoring is still detected after three levels of raising, the bed is gradually laid flat and detection is restarted. Such a cycle is performed up to 3 times (i.e., restoration is performed 3 times). If snoring cannot be stopped after 3 cycles, the snore-stopping function is disabled in a current sleep period until the next use. The above mode may prevent frequent head raising from affecting sleep quality of the user.

In an optional example, the method further includes the following steps: the cumulative raising frequency and the cumulative restoration frequency are reset to zero after collection of the breath sound signals emitted by the object is stopped, and the head of the object is controlled to be restored to the initial height state.

As at least one alternative embodiment, the cumulative raising frequency and the cumulative restoration frequency need to be reset to zero after collection of the breath sound signals emitted by the object is stopped, and the head of the object is restored to the initial height state, such that recounting of a raising frequency and a restoration frequency in the next use is facilitated.

As at least one alternative embodiment, collection of the breath sound signals emitted by the object is stopped after the cumulative raising frequency reaches the preset raising frequency and the cumulative restoration frequency reaches the preset restoration frequency. For instance, if snoring cannot be stopped after 3 cycles, the snore-stopping function is disabled in the current sleep period until the next use, the cumulative raising frequency and the cumulative restoration frequency are reset to zero, and the head of the object is controlled to be restored to the initial height state. If snoring is effectively stopped within 3 cycles, a current height is maintained to complete a current snore-stopping process. If snoring is detected again later in a current sleep period, a last snore-stopping action is continued. That is, the snore-stopping action is performed continuously from a height maintained upon the completion of the last snore-stopping action. If the cumulative raising frequency of upon the completion of the last snore-stopping action reaches the preset raising frequency, that is, a raising height of the head of the object upon the completion of the last snore-stopping action reaches a maximum limit, the head of the object is restored to the initial height state, and then the snore-stopping action is performed again from the initial height state.

In an optional example, the method further includes the following step: in a case that the snore signals are detected in the breath sound signals collected by the sound signal collection device, the detected snore signals are uploaded to a target application in a terminal device.

As at least one alternative embodiment, after the snore signals are detected, the detected snore signals may be uploaded to the target application, and the user may log in to the target application to check a snoring condition. The target application may further generate a snore detection report based on the received snore signals.

Through S102 to S106, smart bed control and snore stopping may be implemented based on the snore signals of the user. In this way, technical effects of effective snore stopping of the user through control of the smart bed, improvement in sleep quality of the user, and better user experience are achieved. In addition, the technical problem that smart beds in the related art do not have a snore-stopping function, resulting in poor user experience and sleep quality is solved.

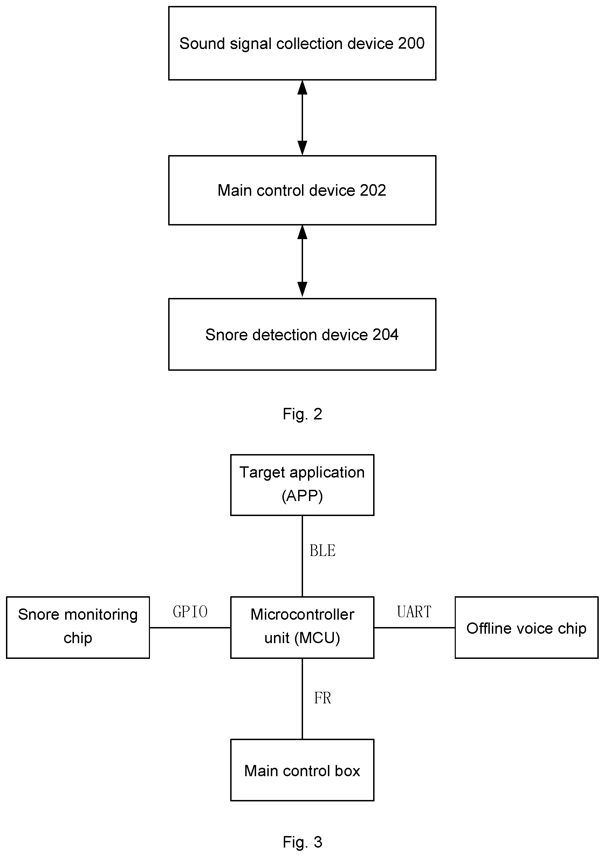

An example of the present disclosure further provides a system example for implementing the method for controlling the smart bed. Fig. 2 is a schematic structural diagram of a system for controlling the smart bed according to an example of the present disclosure. As shown in Fig. 2, the system for controlling the smart bed includes: a sound signal collection device 200, a main control device 202, and a snore detection device 204. The sound signal collection device 200 and the snore detection device 204 are separately connected to the main control device 202. The main control device 202 is configured to perform the method for controlling a smart bed according to any one of the examples.

As at least one alternative embodiment, the main control device may include a microcontroller unit and a main control box. The microcontroller unit is connected to the main control box. The main control device is configured to perform the method for controlling the smart bed according to any one of the examples. The main control box is configured to control an action of the head of the object.

As at least one alternative embodiment, the sound signal collection device may be an offline voice chip. The snore detection device may be a snore monitoring chip. The microcontroller unit, the offline voice chip and the snore monitoring chip may be integrated on a same circuit board or arranged on different circuit boards. Fig. 3 is a schematic structural diagram of an optional system for controlling the smart bed according to an example of the present disclosure. As shown in Fig. 3, the system for controlling the smart bed includes a snore monitoring chip, a microcontroller unit (MCU), an offline voice chip, a main control box, and a target application (APP). The microcontroller unit (MCU) is connected to the target application through Bluetooth low energy (BLE), connected to the offline voice chip through a universal asynchronous receiver/transmitter (UART), connected to the main control box through radio frequency (RF), and connected to the snore monitoring chip through general purpose input/output (GPIO). A smart bed control process implemented based on the system for controlling the smart bed is as follows:
The microcontroller unit (MCU) performs data transmission with the main control box through RF wireless communication, and sends received breath sound signals to the main control box, such that a bed body can be controlled through a plurality of methods. The offline voice chip, the snore monitoring chip and a snore-stopping control action chip may be integrated on one circuit board as accessories, and are in communication with the main control box through RF.

The offline voice chip analyzes the breath sound signals of a user (i.e., an object) through an algorithm, to detect whether snoring occurs, that is, whether snore signals are detected in the breath sound signals. If snoring is detected, the offline voice chip sends a snore signal to the microcontroller unit through pin level transition. After receiving the snore signal, the microcontroller unit stores the signal in a flash memory Flash, and then uploads the signal to the target application (APP) in a terminal device, such that the user may check a snoring condition during sleep through the APP. Every 1 minute, the system analyzes whether snore signals exist in breath sound signals collected within the current 1 minute, records breath sound signals within latest 5 minutes, and calculates whether the snore signals are detected 3 or more times within the 5 minutes. If yes, i.e., the snore signals are detected 3 or more times, a snore-stopping function is enabled. The microcontroller unit sends an instruction to the main control box through RF wireless communication, to raise a head of the user so as to relieve snoring. If the snore signals are not detected 3 or more times within the 5 minutes, it is determined that snore stopping is successful, snoring information of the latest 5 minutes is deleted, and recording of snoring information is restarted. The system may be connected to an APP of the terminal device (for instance, a mobile phone) of the user through BLE, and upload snore signals in Flash to the APP. In this way, the user may see a snoring condition during sleep through the APP and operate an APP interface to achieve functions of the smart bed.

A snore-stopping action includes raising the head of the user (a raising mode is determined according to a bed structure, such as a drive motor or an air bag). A raising height may be selected by the user on the APP (or may be a default value if no selection is made). When a snore signal is detected, the head is raised by one level. If snoring is still detected, the head is raised by another level, with a maximum of three levels in total. If snoring is still detected after three levels of raising, the bed is gradually laid flat for re-detection. Such a cycle may be repeated up to 3 times. If snoring cannot be stopped after 3 cycles, the snore-stopping function is disabled in a current sleep period until the next use. If snoring is effectively stopped within 3 cycles, a current height is maintained to complete a current snore-stopping process.

If snoring is detected again later in a current sleep period, a last snore-stopping action is continued. That is, the snore-stopping action is performed continuously from a height maintained upon the completion of the last snore-stopping action. If the cumulative raising frequency of upon the completion of the last snore-stopping action reaches the preset raising frequency, that is, a raising height of the head of the object upon the completion of the last snore-stopping action reaches a maximum limit, the head of the object is restored to the initial height state, and then the snore-stopping action is performed again from the initial height state.

A process of recording 5-minute snoring information is as follows: 5 pieces of data caches are provided, and one group of breath sound signals are collected every minute. A first-in-first-out mechanism is used. When a new group of breath sound signals are stored, the earliest stored group is removed. Whether to trigger a snore-stopping action or confirm successful snore-stopping is determined by detecting whether 3 or more groups of snore signals exist in the 5 groups of breath sound signals. After the snore-stopping action is performed, all the 5 pieces of cached data are deleted. If snore stopping is determined to be successful, the cached data is not deleted, and detection is continued.

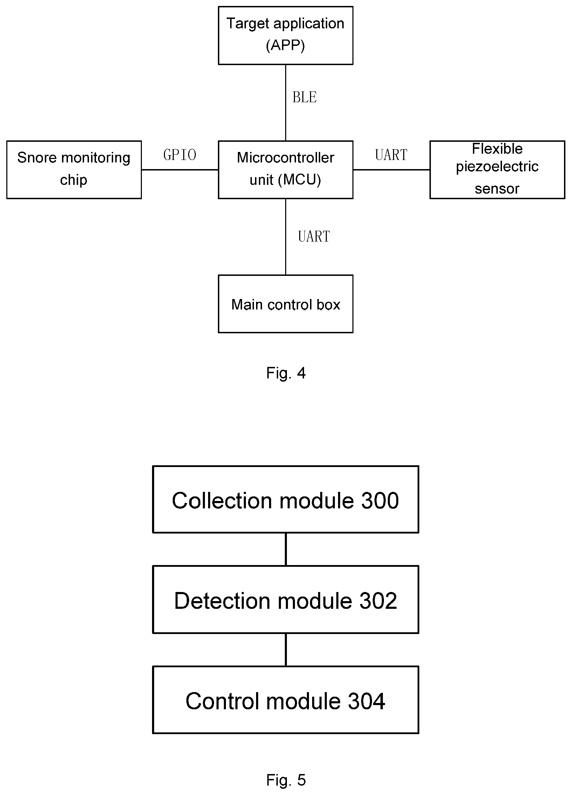

As at least one alternative embodiment, the system for controlling a smart bed may further include a flexible piezoelectric sensor. The flexible piezoelectric sensor may be arranged on a mattress of the smart bed, to form a flexible piezoelectric sensing mattress. The flexible piezoelectric sensor may be configured to monitor an in-bed state and sleep state data of the user, so as to determine whether to enable a sound signal collection function for user snore detection. Fig. 4 is a schematic structural diagram of another optional system for controlling a smart bed according to an example of the present disclosure. As shown in Fig. 4, the system for controlling a smart bed includes a microcontroller unit (MCU), a snore detection sensor, a flexible piezoelectric sensor, a main control box, and a target application (APP). The snore detection sensor integrates a snore monitoring chip and an offline voice chip. The microcontroller unit (MCU) is connected to the target application through BLE, connected to the flexible piezoelectric sensor through a UART, connected to the main control box through the UART, and connected to the snore detection sensor through GPIO. A smart bed control process implemented based on the system for controlling a smart bed is as follows:
For a voice snore monitoring part, the offline voice chip in a voice snore detection sensor analyzes the breath sound signals of the user through an algorithm, to detect whether snoring occurs. If snoring is detected, the offline voice chip sends a snore signal to the microcontroller unit through pin level transition. After the microcontroller unit receives the snore signal, pre-snoring mark data of the current 1 minute is set to 1.

The flexible piezoelectric sensing mattress transmits a heart rate, respiration, an in-bed state and sleep state data to the microcontroller unit through a serial port every minute. When the microcontroller unit determines through parsing from the received data that the user is in an in-bed and falling-asleep state and the pre-snoring mark data of the current 1 minute is 1, current snoring data is recorded as 1. Otherwise, the data is recorded as 0, and breath sound signals of the latest 5 minutes in total are cached.

Whether the snore signals are detected 3 or more times in the breath sound signals within the 5 minutes, if yes, i.e., the snore signals are detected 3 or more times, a snore-stopping action is started. The microcontroller unit sends an instruction to the main control box through serial port communication, to raise a head of the user so as to relieve snoring. If the snore signals are not detected 3 or more times within the 5 minutes, it is determined that snore stopping is successful, the breath sound signals of the latest 5 minutes are deleted, and recording of the breath sound signals is restarted. If the microcontroller unit parses an off-bed state from the received data and determines that the state lasts for a predetermined period (for instance, 10 minutes), snore-stopping action data is deleted, and the head of the user is laid flat.

A snore-stopping action includes raising the head of the user (a raising mode is determined according to a bed structure, such as a drive motor or an air bag). A raising height may be selected by the user on the APP (or may be a default value if no selection is made). When a snore signal is detected, the head is raised by one level. If snoring is still detected, the head is raised by another level, with a maximum of three levels in total. If snoring is still detected after three levels of raising, the bed is gradually laid flat for re-detection. Such a cycle may be repeated up to 3 times. If snoring cannot be stopped after 3 cycles, the snore-stopping function is disabled in a current sleep period until the next use. If snoring is effectively stopped within 3 cycles, a current height is maintained to complete a current snore-stopping process. If snoring is detected again later in a current sleep period, a last snore-stopping action is continued. That is, the snore-stopping action is performed continuously from a height maintained upon the completion of the last snore-stopping action. If the cumulative raising frequency of upon the completion of the last snore-stopping action reaches the preset raising frequency, that is, a raising height of the head of the object upon the completion of the last snore-stopping action reaches a maximum limit, the head of the object is restored to the initial height state, and then the snore-stopping action is performed again from the initial height state.

A process of recording 5-minute snoring information is as follows: 5 pieces of data cach are provided, and one group of breath sound signals is collected every minute. A first-in-first-out mechanism is used. When a new group of breath sound signals are stored, the earliest stored group is removed. Whether to trigger a snore-stopping action or confirm successful snore-stopping is determined by detecting whether 3 or more groups of snore signals exist in the 5 groups of breath sound signals. After the snore-stopping action is performed, all the 5 pieces of cached data are deleted. If snore stopping is determined to be successful, the cached data is not deleted, and detection is continued.

The microcontroller unit further stores minute-by-minute data of a heart rate, respiration, an in-bed state, a sleep state and a snoring state into Flash. The system may be connected to an APP of a mobile phone of the user through BLE, and upload the data in Flash to the APP. In this way, the user may check a sleep state through the APP and operate an APP interface to achieve functions of the smart bed.

It should be noted that specific structures of the system for controlling a smart bed in Fig. 2 to Fig. 4 of the present disclosure are only illustrative. In practical application, the system for controlling a smart bed in the present disclosure may have more or fewer structures than those of the system for controlling a smart bed in Fig. 2 to Fig. 4.

It should be noted that any optional or preferred method for controlling a smart bed in the above method examples may be performed or implemented by the system for controlling a smart bed in the example.

The example further provides a smart bed. The smart bed includes a bed body, and the system for controlling a smart bed. The system for controlling a smart bed is arranged in the bed body.

In addition, it should be noted that reference may be made to the related description in the method example for optional or preferred embodiments of the example, which is not repeated herein.

The example further provides an apparatus for controlling a smart bed. The apparatus is configured to implement the above examples and preferred embodiments, and will not be repeated herein. The terms "module" and "apparatus" as used below may implement a combination of software and/or hardware having predetermined functions. While the apparatus described in the following examples is preferably implemented in software, implementation in hardware or a combination of software and hardware is also possible and conceivable.

An example of the present disclosure further provides an apparatus example for implementing the method for controlling a smart bed. Fig. 5 is a schematic structural diagram of an apparatus for controlling a smart bed according to an example of the present disclosure. As shown in Fig. 5, the apparatus for controlling a smart bed includes: a collection module 300, a detection module 302, and a control module 304.

The collection module 300 is configured to collect, by a sound signal collection device, N groups of breath sound signals emitted by an object at preset time intervals. The object is on the smart bed.

The detection module 302 is connected to the collection module 300 and configured to detect, by a snore detection device, whether snore signals exist in the N groups of breath sound signals.

The control module 304 is connected to the detection module 302 and configured to control the smart bed to perform a snore-stopping action in a case that the number of groups of breath sound signals including the snore signals in the N groups of breath sound signals is detected to be greater than M. M is an integer less than or equal to N.

In the examples of the present disclosure, the collection module 300 is configured to collect, by the sound signal collection device, the N groups of breath sound signals emitted by the object at every preset time interval. The object is on the smart bed. The detection module 302 is connected to the collection module 300 and configured to detect, by the snore detection device, whether the snore signals exist in the N groups of breath sound signals. The control module 304 is connected to the detection module 302 and configured to control the smart bed to perform the snore-stopping action in a case that the number of groups of breath sound signals including the snore signals in the N groups of breath sound signals is detected to be greater than M. M is an integer less than or equal to N. In this way, smart bed control and snore stopping are implemented based on the snore signals of a user. Thus, technical effects of effective snore stopping of the user through control of the smart bed, improvement in sleep quality of the user, and better user experience are achieved. In addition, the technical problem that smart beds in the related art do not have a snore-stopping function, resulting in poor user experience and sleep quality is solved.

It should be noted that the above modules may be implemented in software or hardware, and for instance, implementation in hardware may be achieved through the following method: the above modules may be located in a same processor; or the above modules may be located in different processors in any combination form.

It should be noted herein that the collection module 300, the detection module 302 and the control module 304 correspond to S102 to S106 in the examples. Implementation instances and application scenes of the above modules are consistent with those of the corresponding steps, and are not limited by the content disclosed in the examples. It should be noted that the above modules may be operated in a computer terminal as a part of the apparatus.

It should be noted that reference may be made to the related description in the example for optional or preferred embodiments of the example, which is not repeated herein.

The apparatus for controlling a smart bed may further include a processor and a memory. The collection module 300, the detection module 302 and the control module 304, as program modules, are all stored in the memory. The program modules stored in the memory are executed by the processor to achieve corresponding functions.

The processor includes a core, and the core invokes corresponding program modules in the memory. One or more cores may be provided. The memory may be a volatile memory, a random access memory (RAM), and/or a non-volatile memory, etc., such as a read-only memory (ROM) or a flash RAM, in a computer-readable medium, and the memory includes at least one memory chip.

An example of the present disclosure further provides an example of a non-volatile storage medium. As at least one alternative embodiment, in the example, the non-volatile storage medium includes a stored program. The program controls, during running, a device where the non-volatile storage medium is located to perform the method for controlling a smart bed according to any one of the examples.

As at least one alternative embodiment, in the example, the non-volatile storage medium may be located in any computer terminal in a computer terminal group in a computer network, or in any mobile terminal in a mobile terminal group. The non-volatile storage medium includes a stored program.

As at least one alternative embodiment, the program controls, during running, the device where the non-volatile storage medium is located to perform the following functions: a sound signal collection device collects N groups of breath sound signals emitted by an object at every preset time interval, where the object is on a smart bed; a snore detection device detects whether snore signals exist in the N groups of breath sound signals; and the smart bed is controlled to perform a snore-stopping action in a case that the number of groups of breath sound signals including the snore signals in the N groups of breath sound signals is detected to be greater than M. M is an integer less than or equal to N.

An example of the present disclosure further provides an example of a processor. As at least one alternative embodiment, in the example, the processor is configured to run a program. The program performs, during running, the method for controlling a smart bed according to any one of the examples.

An example of the present disclosure further provides an example of a computer program product. When executed on a data processing device, the computer program product is adapted to execute a program initialized with the steps of the method for controlling a smart bed according to any one of the examples.

As at least one alternative embodiment, when executed on a data processing device, the computer program product is adapted to execute a program initialized with the following method steps: a sound signal collection device collects N groups of breath sound signals emitted by an object at every preset time interval, where the object is on a smart bed; a snore detection device detects whether snore signals exist in the N groups of breath sound signals; and the smart bed is controlled to perform a snore-stopping action in a case that the number of groups of breath sound signals including the snore signals in the N groups of breath sound signals is detected to be greater than M. M is an integer less than or equal to N.

An example of the present disclosure provides an electronic device. The electronic device includes a processor, a memory, and a program stored in the memory and runnable on the processor. When executing the program, the processor implements the following steps: a sound signal collection device collects N groups of breath sound signals emitted by an object at every preset time interval, where the object is on a smart bed; a snore detection device detects whether snore signals exist in the N groups of breath sound signals; and the smart bed is controlled to perform a snore-stopping action in a case that the number of groups of breath sound signals including the snore signals in the N groups of breath sound signals is detected to be greater than M. M is an integer less than or equal to N.

Sequences of the examples of the present disclosure are only for description and do not indicate advantages and disadvantages of the examples.

In the above examples of the present disclosure, the descriptions of all the examples are emphasized on their respective aspects, and reference may be made to the related descriptions of other examples for parts that are not described in detail in an example.

In a plurality of examples provided by the present disclosure, it should be understood that the disclosed technology may be implemented in other ways. The above apparatus example is merely illustrative. For instance, the division of the above modules may be the division based on logical functions. In actual implementation, there may be other division modes. For instance, a plurality of units or components may be combined or integrated into another system, or some features may be ignored or not executed. In addition, the shown or discussed coupling or direct coupling or communication connection between each other may be indirect coupling or communication connection through some interfaces or modules, or may be in an electrical form or another form.

The module described as a separable part may be physically separated or not, and a part shown as a module may be a physical module or not, which may be located at one place or may be distributed on a plurality of modules. Some or all modules may be selected according to actual needs, to achieve the objective of the solution of the example.

In addition, the functional modules in the examples of the present disclosure may be integrated into one processing module, all the modules may be physically present separately, or two or more modules may be integrated into one module. The above integrated module may be implemented in a form of hardware or a software functional module.

If the integrated module is implemented in the form of the software functional module and sold or used as an independent product, the integrated module may be stored in one computer-readable non-volatile storage medium. Based on such understanding, the technical solution of the present disclosure may be embodied in a form of a software product in essence or in part that contributes to the prior art or in part or whole, and the computer software product is stored in one non-volatile storage medium, and includes a plurality of instructions to make one computer device (which may be a personal computer, a server, a network device, etc.) perform all or some steps of the method of each of the examples of the present disclosure. The above non-volatile storage medium includes: a USB flash disk, a read-only memory (ROM), a random access memory (RAM), a mobile hard disk, a magnetic disk, an optical disk, and other media capable of storing program codes.

What are described above are merely preferred embodiments of the present disclosure. It should be noted that those of ordinary skill in the art can make several improvements without departing from the principle of the present disclosure, and these improvements should fall within the protection scope of the present disclosure.

### Industrial Applicability

A solution provided by examples of the present disclosure may be applied to the field of smart home. In the examples of the present disclosure, a sound signal collection device collects N groups of breath sound signals emitted by an object at every preset time interval, where the object is on a smart bed; a snore detection device detects whether snore signals exist in the N groups of breath sound signals; and the smart bed is controlled to perform a snore-stopping action in a case that the number of groups of breath sound signals including the snore signals in the N groups of breath sound signals is detected to be greater than M. M is an integer less than or equal to N. Thus, technical effects of effective snore stopping of a user through control of the smart bed, improvement in sleep quality of the user, and better user experience are achieved.

## Claims

1. A method for controlling a smart bed, comprising:
collecting, by a sound signal collection device, N groups of breath sound signals emitted by an object at every preset time interval, wherein the object is on the smart bed;
detecting, by a snore detection device, whether snore signals exist in the N groups of breath sound signals; and
controlling the smart bed to perform a snore-stopping action in a case that the number of groups of breath sound signals comprising the snore signals in the N groups of breath sound signals is detected to be greater than M, wherein M is an integer less than or equal to N.

2. The method as claimed in claim 1, wherein after the controlling the smart bed to perform a snore-stopping action in a case that the number of groups of breath sound signals comprising the snore signals in the N groups of breath sound signals is detected to be greater than M, the method further comprises:
continuously detecting one or more times whether snore signals exist in breath sound signals collected by the sound signal collection device, and continuously controlling, in a case that results of one or more times of repeated detection indicate that the snore signals exist in the breath sound signals, a head of the object to be further raised one or more times until no snore signals are detected in breath sound signals collected by the sound signal collection device.

3. The method as claimed in claim 2, further comprising:
determining a cumulative raising frequency of the head of the object;
detecting whether the cumulative raising frequency reaches a preset raising frequency; and
controlling the head of the object to be restored to an initial height state in a case that the cumulative raising frequency reaches the preset raising frequency, and controlling the head of the object to be raised again from the initial height state.

4. The method as claimed in claim 3, wherein the controlling the head of the object to be restored to an initial height state in a case that the cumulative raising frequency reaches the preset raising frequency, and controlling the head of the object to be raised again from the initial height state comprise:
determining a cumulative restoration frequency of the head of the object in a case that the cumulative raising frequency reaches the preset raising frequency, wherein the cumulative restoration frequency is configured to indicate a frequency of restoring the head of the object to the initial height state; and
controlling the head of the object to be restored to the initial height state in a case that the cumulative restoration frequency does not reach a preset restoration frequency, and controlling the head of the object to be raised again from the initial height state.

5. The method as claimed in claim 4, further comprising:
stopping collection of breath sound signals emitted by the object in a case that the cumulative restoration frequency reaches the preset restoration frequency.

6. The method as claimed in claim 5, further comprising:
resetting the cumulative raising frequency and the cumulative restoration frequency to zero after stopping collection of the breath sound signals emitted by the object, and controlling the head of the object to be restored to the initial height state.

7. The method as claimed in any one of claims 1 to 6, wherein the collecting, by a sound signal collection device, N groups of breath sound signals emitted by an object at every preset time interval comprises:
determining time during which the object is on the smart bed in a case that the object is detected to be on the smart bed;
detecting whether the time during which the object is on the smart bed falls within a preset period; and
collecting, by the sound signal collection device, the N groups of breath sound signals emitted by the object at every preset time interval in a case that the time during which the object is on the smart bed is detected to fall within the preset period.

8. The method as claimed in any one of claims 1 to 6, wherein the collecting, by a sound signal collection device, N groups of breath sound signals emitted by an object at every preset time interval comprises:
detecting an in-bed state and sleep state data of the object based on a flexible piezoelectric sensor; and
collecting, by the sound signal collection device, the N groups of breath sound signals emitted by the object at every preset time interval in a case that the in-bed state indicates that the object is on the smart bed and the sleep state data indicates that the object is in a sleep state.

9. The method as claimed in any one of claims 1 to 6, further comprising:
uploading, in a case that the snore signals are detected in the breath sound signals collected by the sound signal collection device, the detected snore signals to a target application in a terminal device.

10. The method as claimed in any one of claims 1 to 6, wherein the snore-stopping action at least comprises: controlling the head of the object to be raised to a preset height.

11. A system for controlling a smart bed, comprising a sound signal collection device, a main control device, and a snore detection device, wherein
the sound signal collection device and the snore detection device are separately connected to the main control device, wherein the main control device is configured to perform the method for controlling the smart bed as claimed in any one of claims 1 to 10.

12. A smart bed, comprising a bed body, and the system for controlling the smart bed as claimed in claim 11, wherein the system for controlling the smart bed is arranged in the bed body.

13. An electronic device, comprising one or more processors and a memory, wherein the memory is configured to store one or more programs, and when the one or more programs are executed by the one or more processors, the one or more processors are caused to implement the method for controlling the smart bed as claimed in any one of claims 1 to 10.
